(19) Europäisches Patentamt
European Patent Office
Office européen des brevets

(11) **EP 4 477 109 A1**

(12) **EUROPEAN PATENT APPLICATION**

(43) Date of publication:
**18.12.2024 Bulletin 2024/51**

(21) Application number: **23183884.8**

(22) Date of filing: **06.07.2023**

(51) International Patent Classification (IPC):
*A46B 15/00* (2006.01)    *A61B 5/107* (2006.01)

(52) Cooperative Patent Classification (CPC):
**A46B 15/0006; A61B 5/1071; A61B 5/6898;
A61B 5/7267;** A46B 2200/1066; A61B 2562/0219

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.06.2023 US 202363472569 P**

(71) Applicant: **Koninklijke Philips N.V.
5656 AG Eindhoven (NL)**

(72) Inventors:
• VIJAYKUMAR, Adithya
  Eindhoven (NL)
• VLUTTERS, Ruud
  Eindhoven (NL)
• RIETMAN, Ronald
  Eindhoven (NL)

(74) Representative: **Philips Intellectual Property &
Standards
High Tech Campus 52
5656 AG Eindhoven (NL)**

(54) **ESTIMATING HEAD TILT ANGLE OF TOOTHBRUSH USER**

(57) The subject-matter of the present disclosure relates to a computer-implemented method of estimating a head tilt angle of a user of a toothbrush during brushing. The computer-implemented method comprises: sensing, using a sensor of the toothbrush, movement parameters associated with the toothbrush over a brushing time period; inputting, to a machine learning model, the sensed movement parameters to estimate a segment of a dental arch being contacted by the bristles at each time point of the brushing time period; calculating, using a mathematical heuristics model, an orientation of the toothbrush from the movement parameters at each time point of the brushing time period; and for each time point, calculating the head tilt angle of the user of the toothbrush based on the estimated segment of the dental arch and the calculated orientation.

Figure 4

EP 4 477 109 A1

**Description**

FIELD OF THE INVENTION

The subject-matter of the present disclosure relates to a computer-implemented method of estimating a head tilt angle of a user of a toothbrush during brushing, a computer-implemented method of training a machine learning model to estimate a segment of a dental arch of a user being brushed by a user of a toothbrush, a computer-implemented method of training a machine learning model to estimate a brushing angle between bristles of a toothbrush and teeth of a user of the toothbrush, and a transitory, or non-transitory, computer-readable medium.

BACKGROUND OF THE INVENTION

Modern toothbrushes include a body, a head having bristles, an inertial measurement unit (IMU) in the body, and a controller for monitoring and controlling various features of the toothbrush. For example, a toothbrush may provide an alert when the head requires changing after a certain number of uses, or an alert that the head may need replacing if the toothbrush has been dropped.

Such functions are governed by algorithms stored in a storage of the controller and executed by a processor of the controller. Some of those algorithms require inputs associated with various parameters of the toothbrush. Some inputs can be sensed directly from the IMU, such as angular velocity. However, some parameters are not readily available. For example, head tilt angle of the user is not possible to measure using the IMU.

It is an aim of the subject-matter of the present disclosure to improve on the prior art.

SUMMARY OF THE INVENTION

According to a first aspect of the present invention, there is provided a computer-implemented method of estimating a head tilt angle of a user of a toothbrush during brushing, the computer-implemented method comprising: sensing, using a sensor of the toothbrush, movement parameters associated with the toothbrush over a brushing time period; inputting, to a machine learning model, the sensed movement parameters to estimate a segment of a dental arch being contacted by the bristles at each time point of the brushing time period; calculating, using a mathematical heuristics model, an orientation of the toothbrush from the movement parameters at each time point of the brushing time period; and for each time point, calculating the head tilt angle of the user of the toothbrush based on the estimated segment of the dental arch and the calculated orientation.

Advantageously, the machine learning model enables the head tilt angle to be determined based on readily available movement parameters.

In an embodiment, the method may comprise generating and/or sending a signal indicating the head tilt angle.

In an embodiment, when the head tilt angle is in a nodding direction, and when the estimated segment is at a front of the dental arch, the orientation of the toothbrush is an amount of roll of the toothbrush, wherein, when the head tilt angle is in a nodding direction, and when the estimated segment is at a side of the dental arch, the orientation of the toothbrush is an amount of pitch of the toothbrush, wherein, when the head tilt angle is in a sideways rolling direction, and when the estimated segment is at a front of the dental arch, the orientation of the toothbrush is an amount of pitch of the toothbrush, and wherein, when the head tilt angle is in a sideways rolling direction, and when the estimated segment is at a side of the dental arch, the orientation of the toothbrush is an amount of roll of the toothbrush.

In an embodiment, the machine learning model is a second machine learning model, and wherein the method further comprises: inputting, to a first machine learning model, the sensed movement parameters to estimate a brushing angle between bristles at a head of the toothbrush and teeth of the user at each time point of the brushing time period, and wherein the, for each time point, calculating the head tilt angle of the user of the toothbrush based on the estimated segment of the dental arch and the calculated orientation comprises: subtracting the calculated orientation from the brushing angle.

In an embodiment, the first and the second machine learning models each comprises a long short-term memory recurrent neural network, LSTM.

In an embodiment, the first machine learning model comprises an encoder between an input layer and the LSTM and comprises a decoder between the LSTM and an output layer, wherein the encoder comprises a convolutional neural network, wherein the decoder comprises a convolutional neural network.

In an embodiment, the encoder and the LSTM of the first machine learning model is shared with the second machine learning model. The term "shared" may be understood to mean that the first machine learning model and the second machine learning model each use a common encoder and LSTM. In other words, there is a single encoder and a single LSTM used by both the first machine learning model and the second machine learning model.

In an embodiment, the movement parameters include acceleration and angular velocity and the sensor is an inertial measurement unit.

In an embodiment, movement parameters to estimate a segment of the dental arch being contacted by the bristles at each time point of the brushing time period comprises inputting, to the second machine learning model, the sensed movement parameters to estimate a segment of the dental arch being contact by the bristles from amongst a list a segments of dental arches including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual. The incisors may be an interface between the front and each of the left and right dental arch segments.

According to another aspect of the present disclosure, there is provided a computer-implemented method of training a machine learning model to estimate a segment of a dental arch of a user being brushed by a user of a toothbrush, the computer-implemented method comprising: receiving a training data set and a segment ground truth, wherein, for each time point of a brushing time period, the training data set includes movement parameter values and the segment ground truth includes a label indicating a segment of a dental arch being brushed; estimating, using the machine learning model, a segment of a dental arch for each time point of the brushing time period using the respective movement parameter values as inputs; calculating a loss between the estimated segments and the segments indicated by the labels; and adjusting a parameterisation of the machine learning model to minimize the loss.

In an embodiment, the method comprises generating and/or sending a signal containing the machine learning model.

In an embodiment, the computer-implemented method further comprises: generating the segment ground truth by: capturing a video of a user brushing their teeth using a toothbrush over a sample period, sensing, using a sensor of the toothbrush, movement parameter values at each time point of the sample period, displaying the video to a user of a user interface, receiving a plurality of user inputs indicating a segment of a dental arch currently being brushed by the toothbrush in the video, and labelling each time point of the sample period using the segments from the plurality of user inputs; and sending the ground truth to the machine learning model.

In an embodiment, the machine learning model comprises a long short-term memory recurrent neural network, LSTM.

In an embodiment, the machine learning model comprises an encoder between an input layer and the LSTM and comprises a classifier between the LSTM and an output layer, wherein the encoder comprises a convolutional neural network, and wherein the classifier comprises a convolutional neural network and a softmax layer.

In an embodiment, the movement parameters include acceleration and angular velocity and the sensor is an inertial measurement unit.

In an embodiment, the inputting, to a second machine learning model, the sensed movement parameters to estimate a segment of the teeth being contacted by the bristles at each time point of the brushing time period comprises inputting, to the second machine learning model, the sensed movement parameters to estimate a segment of the teeth being contact by the bristles from amongst a list a segments of teeth including upper front facial, , and upper front lingual, upper right facial, upper right occlusal, upper right lingual, upper left facial, upper left occlusal, upper left lingual, lower front facial, , and lower front lingual, lower right facial, lower right occlusal, lower right lingual, lower left facial, lower left occlusal, lower left lingual.

According to an aspect of the invention, there is provided a computer-implemented method of training a machine learning model to estimate a brushing angle between bristles of a toothbrush and teeth of a user of the toothbrush, the computer-implemented method comprising: receiving a training data set and a brushing angle ground truth, wherein, for each time point of a brushing time period, the training data set includes movement parameter values and the brushing angle ground truth; estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss.

In an embodiment, the method may comprise generating and/or sending a signal containing the machine learning model.

In an embodiment, the computer-implemented method further comprises: generating the brushing angle ground truth by: capturing a video of a user brushing their teeth with a toothbrush over a sample period, the user having a first marker on their forehead and the toothbrush having a second marker on its handle, sensing, using a sensor of the toothbrush, movement parameter values at each time point of the sample period, calculating, at each time point of the sample period, a brushing angle based on relative positions of the first marker and the second marker, and generating, for each time point, a brushing angle ground truth associated with the movement parameter values; and sending the brushing angle ground truth to the machine learning model.

In an embodiment, the calculating a brushing angle based on relative positions of the first marker and the second marker comprises calculating the brushing angle using formula 1 for upper teeth and using formula 2 for lower teeth, wherein formula 1 is $\beta = arctan2((h.n), -(h.z))$, and formula 2 is a $\beta = arctan2((h.n),(h.z))$, where $\beta$ is the brushing angle, h denotes a vector pointing away from a point P along bristles of the toothbrush to the toothbrush, where P denotes a point where the bristles touch the teeth, n denotes a vector extending outwardly normal to a curve of the teeth, and z denotes an upward, in-use, normal to a plane of an upper jaw.

In an embodiment, the calculating a loss between the estimated brushing angles and the brushing angle ground truth comprises, for each time point of the brushing time period, determining a ground truth unit vector, $v_{gt}$, and an estimated brushing angle unit vector, $v_{eba}$, by respectively calculating cosine and sine components of the brushing angles in the ground truth and the estimated brushing angles, and calculating the loss, L, using $L = 1 - v_{gt}. v_{eba}$, where . denotes an inner dot product between vgt and veba.

In an embodiment, the first and the second machine learning models each comprises a long short-term memory recurrent neural network, LSTM.

In an embodiment, the first machine learning model comprises an encoder between an input layer and the LSTM and comprises a decoder between the LSTM and an output layer, wherein the encoder comprises a convolutional neural network, and wherein the decoder comprises a convolutional neural network.

In an embodiment, the movement parameters include acceleration and angular velocity and the sensor is an inertial measurement unit.

In an embodiment, the inputting, to a second machine learning model, the sensed movement parameters to estimate a segment of the teeth being contacted by the bristles at each time point of the brushing time period comprises inputting, to the second machine learning model, the sensed movement parameters to estimate a segment of the teeth being contact by the bristles from amongst a list a segments of teeth including upper front facial, , and upper front lingual, upper right facial, upper right occlusal, upper right lingual, upper left facial, upper left occlusal, upper left lingual, lower front facial, , and lower front lingual, lower right facial, lower right occlusal, lower right lingual, lower left facial, lower left occlusal, lower left lingual.

In an embodiment, the computer-implemented method further comprises: providing the machine learning model trained to estimate a segment of a dental arch of a user being brushed by a user of a toothbrush using the computer-implemented method; re-initialising parameters associated with a final layer of the machine learning model; and fine-tuning the machine learning model by the: estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss. In this way, the machine learning model trained to estimate brushing angle can be trained on less data. This is particularly beneficial here because it is much more difficult to obtain data relating to estimation of brushing angle than it is for estimating the dental arch segment because dental arch segment can be manually labelled outside of a lab setting by individual users with ease.

According to an aspect of the invention, there is provided a transitory, or non-transitory, computer-readable medium having instructions stored thereon that, when executed by a computer, cause the computer to perform the computer-implemented method of any preceding aspect or embodiment.

These and other aspects of the present invention will be apparent from and elucidated with reference to the embodiment(s) described hereinafter.

BRIEF DESCRIPTION OF DRAWINGS

The embodiments of the present invention may be best understood with reference to the accompanying figures, in which:

Fig. 1 shows a schematic block diagram of a toothbrush according to one or more embodiments;

Fig. 2 shows an illustration of upper and lower dental arches of a user of the toothbrush of Fig. 1;

Fig. 3 shows schematic illustrations of the toothbrush from Fig. 1 at different brushing angles, in-use;

Fig. 4 shows a block diagram of a machine learning model trained to estimate brushing angle according to one or more embodiments;

Figs. 5A and 5B shows graphs of brushing angle versus time to compare an estimation of brushing angle from the machine learning model in Fig. 4 and a ground truth used to train the machine learning mode, for two different brushing periods;

Fig. 6 shows a block diagram of a machine learning model trained to estimate a segment of a dental arch being brushed by the toothbrush form Fig. 1 according to one or more embodiments;

Fig. 7 shows a block diagram of a machine learning model including the machine learning model of Fig. 4 and the machine learning model of Fig. 6;

Fig. 8 shows a block diagram of a mathematical heuristics model for calculating a brushing angle according to one or more embodiments;

Fig. 9 shows a graph of brushing angle versus time for a brushing period comparing respective outputs of the machine learning model from Fig. 4 and the mathematical heuristics model from Fig. 8;

Fig. 10 shows a schematic of a set-up of a training laboratory for collecting training data for a ground truth for training the machine learning model from Fig. 4;

Fig. 11 shows a schematic of a toothbrush used in the training laboratory from Fig. 10;

Fig. 12 shows a schematic of a head marker used in the training laboratory from Fig. 10;

Fig. 13 shows a flow chart summarising steps associated with a computer-implemented method of estimating a head tilt angle of a user of a toothbrush during brushing using the machine learning model of Fig. 4 according to one or more embodiments;

Fig. 14 shows a flow chart summarising steps associated with a computer-implemented method of training the machine learning model from Fig. 6 according to one or more embodiments; and

Fig. 15 shows a flow chart summarising steps associated with a computer-implemented method of training the machine learning model of Fig. 4 according to one or more embodiments.

## DESCRIPTION OF EMBODIMENTS

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

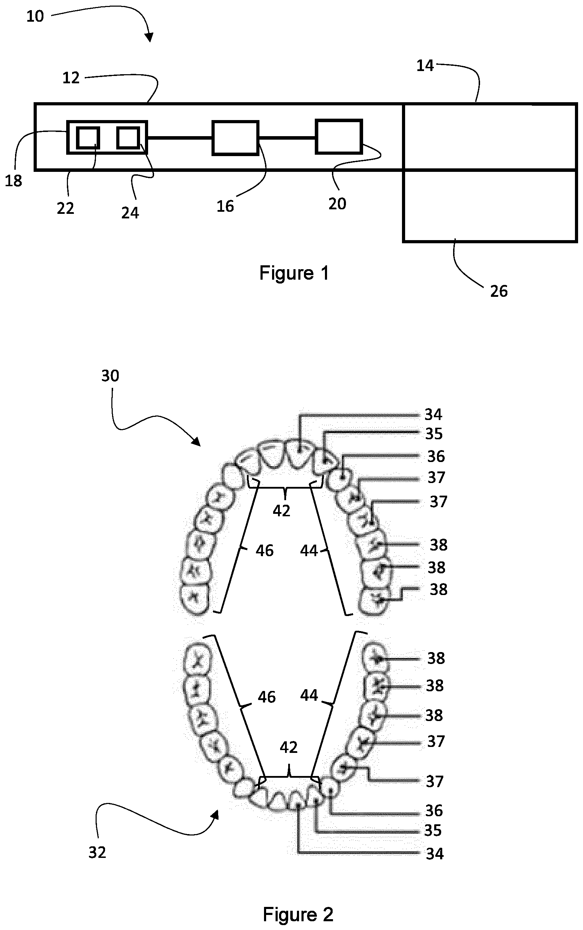

With reference to Fig. 1, a toothbrush 10 includes an elongate body forming a handle 12, and a head 14 at an end of the handle 12. The toothbrush also includes an energy storage module 16, a controller 18, and a sensor 20 within the handle.

The energy storage module 16 may be a battery. The battery may be a secondary, or rechargeable, battery. The controller may include a processor 22 and storage 24. The computer-implemented methods described below may be embodied as electronic data defining instructions stored in the storage as non-transitory computer-readable media that when executed by the processor cause the processor to perform the respective computer-implemented method. The instructions may be loaded onto the storage and thus be embodied as transitory computer readable media.

The sensor 20 may be an inertial measurement unit (IMU) sensor. The IMU sensor may be configured to sense rotational velocity and acceleration. The rotational velocity and acceleration may be sensed in three axes, which may be cartesian axes including x, y, and z axes. The energy storage module 16 may be connected to the controller 18 and the sensor 20 to power them both.

The head 14 may include bristles 26 for brushing a user's teeth.

With reference to Fig. 2, a user's teeth may be categorised into an upper dental arch 30 and a lower dental arch 32. Each of the upper and lower dental arches should include the following tooth types: central incisor 34, lateral incisor 35, canine 36, premolar 37, and molar 38. Each tooth type other than the central and lateral incisors 34, 35, includes a facial side, a lingual side, and an occlusal side. The central and lateral incisors include a facial side and a lingual side, yet no occlusal side.

The dental arches may be split into different segments. The tooth types may be grouped within the segments. There may be sixteen segments. There may be eight segments in the upper dental arch 30 and eight segments in the lower dental arch 32. The segments may include two segments at a front 42 of each dental arch, three segments at a right 44 side of each dental arch, and three segments at a left 46 side of each dental arch. The central and lateral incisors 34, 35, are within the front 42 of each dental arch. The interface between the incisors (e.g. the lateral incisors) and the canines 36 may be the interface between the front and left/right 42, 44 segments.

In this way, the segments include upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, and lower left lingual.

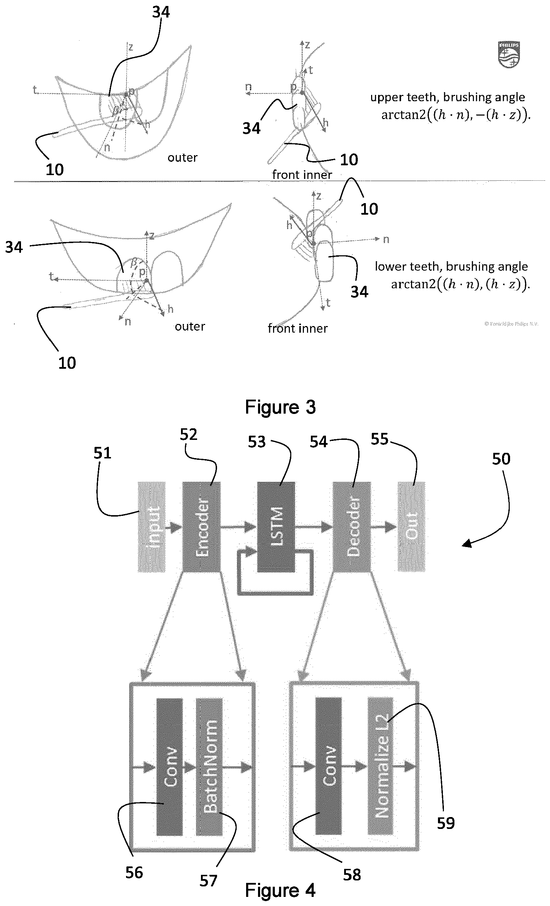

In other embodiments, there may be a different number of segments, and the segments may include a different number of teeth. For example, there may be a segment definition where each segment includes a single tooth.
Before describing the machine learning models, it is worthwhile defining what is meant by brushing angle, β.
With reference to Fig. 3, the brushing angle is calculated using formula 1 for teeth of the upper dental arch and using formula 2 for teeth of the lower dental arch,

wherein formula 1 is

$$\beta = arctan2\big((h.n), -(h.z)\big),$$

and

formula 2 is a

$$\beta = arctan2\big((h.n), (h.z)\big),$$

where β is the brushing angle, h denotes a vector pointing away from a point P along bristles of the toothbrush to the toothbrush, where P denotes a point where the bristles touch the teeth, n denotes a vector extending outwardly normal to a curve of the teeth, and z denotes an upward, in-use, normal to a plane of an upper jaw.

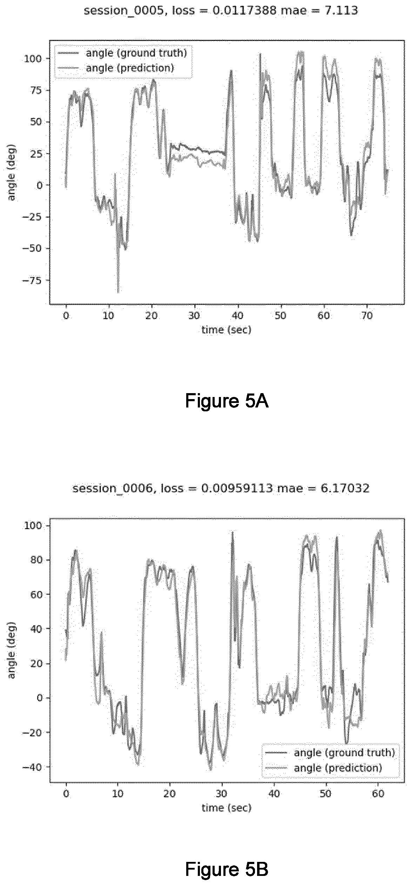

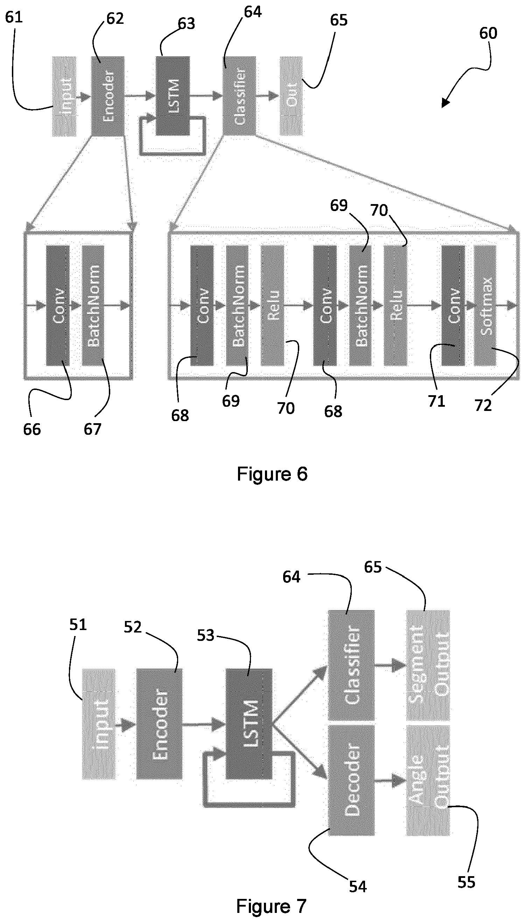

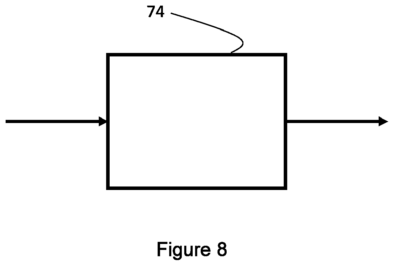

With reference to Fig. 4, a machine learning model is provided for estimating the brushing angle between the bristles and teeth of the user at each time point of a brushing time period using sensed movement parameters as inputs. This machine learning model may be called a first machine learning model 50.
The first machine learning model may include a recurrent neural network, which may be a long short-term memory recurrent neural network, LSTM. The first machine learning model 50 comprises an input 51, an encoder 52, the LSTM 53, a decoder 54, and an output 55. The encoder 52 includes a convolutional neural network 56 (or convolution layer) and a BatchNorm layer 57. The decoder 54 includes a convolutional neural network 58 (or convolution layer) and a normalise L2 layer 59.
In the encoder 52, the input movement parameters get filtered (linearly) by the convolution layer and scaled by the batch normalization layer. This filtered signal goes into the LSTM layer that updates its internal (also called hidden) state. Next, the hidden state of the LSTM is used as input to the decoder, that maps the hidden state into two channels (using a convolution layer with 2 output channels, and a kernel size of 1). As last step, these 2 channels are normalized to represent a vector of length 1.
The method for training the first machine learning model is described in more detail below. However, for reference, Figs. 5A and 5B each show comparisons of brushing angle estimations obtained from the first machine learning model and the ground truth used to train it for two different brushing time periods.
With reference to Fig. 6, a second machine learning model 60 is provided for estimating a segment of a dental arch being contacted by the bristles at each time point of the brushing time period using the movement data as inputs.
The second machine learning model 60 includes an input 61, an encoder 62, an LSTM 63, a classifier 64, and an output 65. The encoder 62 includes a convolutional neural network 66 (or convolution layer) and a BatchNorm layer 67. The classifier 64 includes a first group of three layers including a convolutional neural network 68 (convolution layer), a BatchNorm layer 69, and an activation function 70, and a second group including the same three layers in the same order as the first group. The activation function 70 may be a ReLu activation function. The classifier 64 also includes a convolutional neural network 71 (or convolution layer) and a softmax layer 72. The softmax layer includes a plurality of nodes representing a probability distribution, where a total of the probabilities of the probabilities of all of the plurality of nodes equals 1.
With reference to Fig. 7, in some embodiments, the first machine learning model and the second machine learning model may share an input 51, an encoder 52, and an LSTM 53. The decoder 54 and angle output 55 may form a first branch after the LSTM 53. The classifier 64 and the segment output 65 may form a second branch after the LSTM 53. In this way, there can be a reduction in the amount of computation required when running the first and second machine learning models.
With reference to Fig. 8, a mathematical heuristics model 74 calculates an orientation of the toothbrush from the movement parameters at each time point of the brushing time period. The term orientation may be used to mean brushing angle, which may be understood in terms of pitch or roll of the toothbrush.
With reference to Fig .9, outputs of the mathematical heuristics model 74 and the first machine learning model 50 can be seen to provide very similar estimations for brushing angle when there is no head tilt but provide different estimations for brushing angle when head tilt is present. This fact can be exploited so as to calculate a head tilt angle of the user of the toothbrush. More specifically, during inference time, for each time point, the head tilt angle of the user of the toothbrush may be calculated by subtracting the calculated orientation from the brushing angle. This difference may be calculated using formula 3 below.

$$\text{Formula 3} \qquad \text{head tilt angle= Abs(M\_AI(IMU) – M\_mat(IMU))},$$

Where Abs represents the absolute value, M_AI(IMU) is the estimated brushing angle from the first machine learning mode based on the movement parameters from the IMU used as inputs, and M_mat(IMU) is the calculated orientation of using the mathematical heuristics model using the movement parameters from the IMU as inputs.

It should be noted that in certain embodiments, the first machine learning model may not be needed since it is possible to calculate the head tilt angle directly from the mathematical heuristics model using certain rules. For instance, when the head tilt angle is in a nodding direction, and when the estimated segment is at a front of the dental arch, the orientation of the toothbrush is an amount of roll of the toothbrush, wherein, when the head tilt angle is in a nodding direction, and when the estimated segment is at a side of the dental arch, the orientation of the toothbrush is an amount of pitch of the toothbrush, wherein, when the head tilt angle is in a sideways rolling direction, and when the estimated segment is at a front of the dental arch, the orientation of the toothbrush is an amount of pitch of the toothbrush, and wherein, when the head tilt angle is in a sideways rolling direction, and when the estimated segment is at a side of the dental arch, the orientation of the toothbrush is an amount of roll of the toothbrush.

In this way, for each time point, the head tilt angle of the user of the toothbrush is calculated based on the estimated segment of the dental arch and the calculated orientation.

To train the first and second machine learning models, first the second machine learning model is trained.

The segment ground truth may be generated by capturing a video of a user brushing their teeth using a toothbrush over a sample period, sensing, using a sensor of the toothbrush, movement parameter values at each time point of the sample period, displaying the video to the user of the toothbrush using a user interface, receiving a plurality of user inputs indicating a segment of a dental arch currently being brushed by the toothbrush in the video, and the user of the toothbrush then manually labelling each time point of the sample period using the segments from the plurality of user inputs. The IMU data, or movement parameters of each time point, may be taken as a training data set and the ground truth include the labels indicating the segment of the dental arch being brushed.

The method of training the machine learning model includes receiving the training data set and the segment ground truth, estimating, using the machine learning model, a brushing segment for each time point of the brushing time period using the respective movement parameter values as inputs, calculating a loss between the estimated brushing segments and the segments indicated by the labels, and adjusting a parameterisation of the machine learning model to minimise the loss. The adjusting the parameters may be carried out using an optimisation algorithm. The parameterisation may refer to weights throughout the machine learning model.

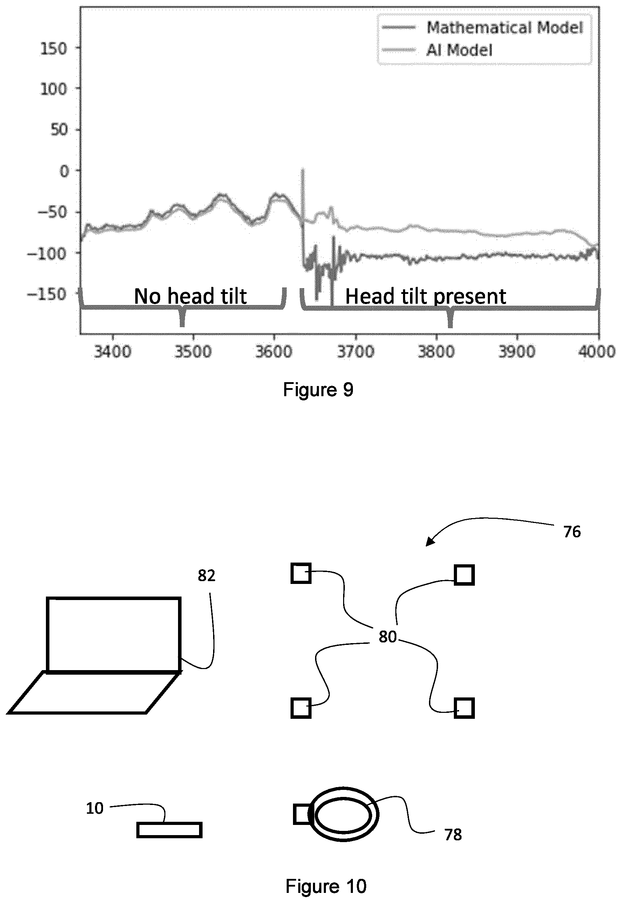

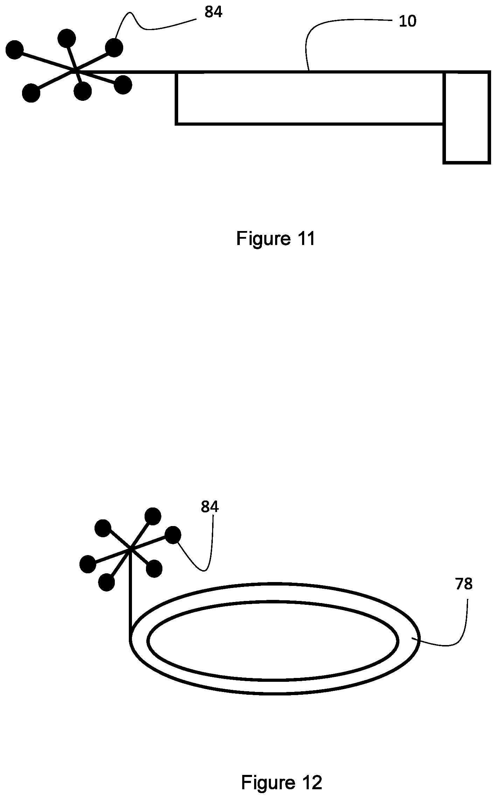

With reference to Figs. 10 to 12, another aspect relates to a computer-implemented method of training the first machine learning model to estimate a brushing angle between bristles of the toothbrush and teeth of the user of the toothbrush. The method first requires collection of training data and generating a corresponding ground truth. The training data may be collected in a laboratory 76 where the ground truth is constructed.

The laboratory includes a toothbrush 10, a headset 78, cameras 80, and a computing system 82. The toothbrush 10 includes a plurality of markers 84 fixed thereto. The markers may be detected by the cameras 80 so that an orientation of the toothbrush can be detected. The headset may also include a plurality of markers 86 which may be detected by the cameras 80 to deduce a head tilt angle of the user.

The method may comprise, generating the brushing angle ground truth by capturing a video of a user brushing their teeth with a toothbrush over a sample period, the user having a first marker on their forehead and the toothbrush having a second marker on its handle, sensing, using a sensor of the toothbrush, movement parameter values at each time point of the sample period, calculating, at each time point of the sample period, a brushing angle based on relative positions of the first marker and the second marker, and generating, for each time point, a brushing angle ground truth associated with the movement parameter values; and sending the brushing angle ground truth to the machine learning model.

The method comprises receiving the training data set and the brushing angle ground truth, wherein, for each time point of a brushing time period, the training data set includes movement parameter values and the brushing angle ground truth includes a brushing angle ground truth; estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss. The adjustment may be performed using an optimisation algorithm, and the parameterisation may refer to weights of the machine learning model.

The calculating a loss between the estimated brushing angles and the brushing angle ground truth, for each time point of the brushing time period, determining a ground truth unit vector, $v_{gt}$, and an estimated brushing angle unit vector, $v_{eba}$, by respectively calculating cosine and sine components of the brushing angles in the ground truth and the estimated brushing angles, and calculating the loss, L, using $L = 1 - v_{gt} \cdot v_{eba}$ where . denotes an inner dot product between $v_{gt}$ and $v_{eba}$.

With further reference to Fig. 3, in other words, based on the angle definition/interpretation, the next 3 categories of user movements when brushing a specific tooth can be defined.

In a first category, the toothbrush rotates around the t-axis (gumline). Angle detection and coaching focuses on coaching such motion to stay optimally at 45 degrees Bass-angle.

In a second category, the toothbrush moves on the tooth plane. This kind of motion can be typically caused by holding the brush more vertically than horizontally. To promote the maximal contact with jawline, users should be promoted to hold the brush horizontally.

In a third category, the toothbrush moves on the toothbrush plan. This kind of motion does not seem to be natural because the toothbrush moves away from the tooth surface, but it may happen when the toothbrush moves from the next tooth.

Movements in categories 1) and 2) can be monitored and coached.

In the end, one can calculate the brushing angle $\beta$ for each timestep (typically 1/30 sec) of the brushing session and save this in combination with the IMU sensor data.

During the training procedure, the IMU data will be used as input, and the brushing angle as ideal / ground-truth that we want to obtain. Hence, when an AI model predicts the brushing angle, one can use this ideal brushing angle with the predicted brushing angle and minimize the difference. As it would be desirable to predict angles, one should ensure that the metric being minimized (also called the loss function) should converge nicely. When naively using a simple mean absolute difference in angles, one might suffer from the circular definition of these angles, where an angle of -179 is only 2 degrees away from an angle of + 179 degree. To solve this, it is possible to predict the cosine and sine components of the brushing angle (effectively a vector with length = 1) and convert the ground truth also to the cosine and sine of the ground truth angle.

It should be noted that whilst it is possible to train the first machine learning model using randomised initialisation of weights, it may be better in certain circumstances to start with the trained second machine learning model and use transfer learning. This because the ground truth and raining data for training the second machine learning model does not have to be carried out in the laboratory. Instead of the cameras using the laboratory, a smart phone of a user can be used outside the context of the laboratory.

To train the first machine learning model the method may comprise providing the second machine learning model trained using the computer-implemented method described above; re-initialising parameters associated with a final layer of the machine learning model; and fine-tuning the machine learning model by the: estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss.

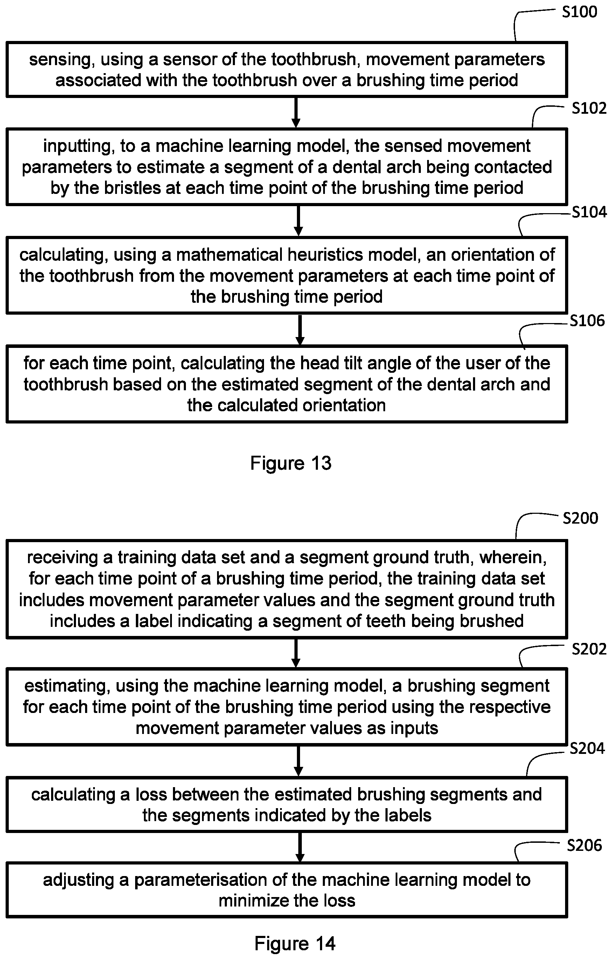

With reference to Fig. 13, the computer-implemented method of estimating a head tilt angle of a user of a toothbrush during brushing may be summarised as including: sensing S 100, using a sensor of the toothbrush, movement parameters associated with the toothbrush over a brushing time period; inputting S 102, to a machine learning model, the sensed movement parameters to estimate a segment of a dental arch being contacted by the bristles at each time point of the brushing time period; calculating S 104, using a mathematical heuristics model, an orientation of the toothbrush from the movement parameters at each time point of the brushing time period; and for each time point, calculating S 106 the head tilt angle of the user of the toothbrush based on the estimated segment of the dental arch and the calculated orientation.

With reference to Fig. 14, the computer-implemented method of training a machine learning model to estimate a segment of teeth of a user being brushed by a user of a toothbrush may be summarised as including: receiving S200 a training data set and a segment ground truth, wherein, for each time point of a brushing time period, the training data set includes movement parameter values and the segment ground truth includes a label indicating a segment of teeth being brushed; estimating S202, using the machine learning model, a brushing segment for each time point of the brushing time period using the respective movement parameter values as inputs; calculating S204 a loss between the estimated brushing segments and the segments indicated by the labels; and adjusting S206 a parameterisation of the machine learning model to minimize the loss.

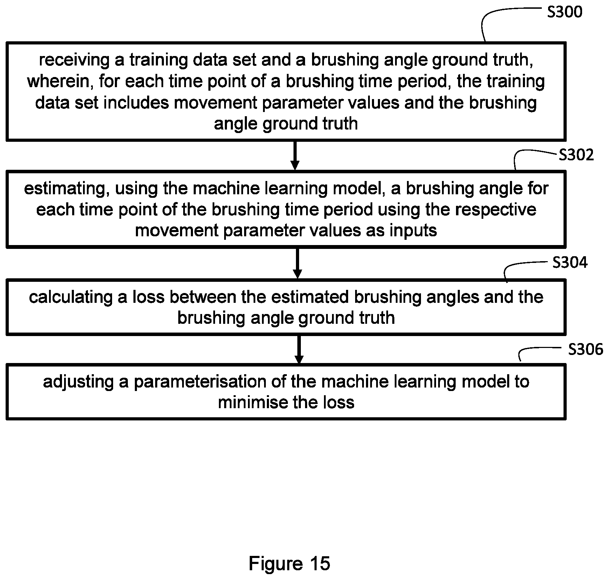

With reference to Fig. 15, the computer-implemented method of training a machine learning model to estimate a brushing angle between bristles of a toothbrush and teeth of a user of the toothbrush may be summarised as including: receiving S300 a training data set and a brushing angle ground truth, wherein, for each time point of a brushing time period, the training data set includes movement parameter values and the brushing angle ground truth; estimating S302, using the machine learning model, a brushing angle for each time point of the brushing time period using the respective movement parameter values as inputs; calculating S304 a loss between the estimated brushing angles and the brushing angles ground truth; and adjusting S306 a parameterisation of the machine learning model to minimise the loss.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

Various features disclosed herein may be summarised in the following clauses.

Clause 1. A computer-implemented method of estimating a head tilt angle of a user of a toothbrush during brushing, the computer-implemented method comprising: sensing, using a sensor of the toothbrush, movement parameters associated with the toothbrush over a brushing time period; inputting, to a machine learning model, the sensed movement parameters to estimate a segment of a dental arch being contacted by the bristles at each time point of the brushing time period; calculating, using a mathematical heuristics model, an orientation of the toothbrush from the movement parameters at each time point of the brushing time period; and for each time point, calculating the head tilt angle of the user of the toothbrush based on the estimated segment of the dental arch and the calculated orientation.

Clause 2. The computer-implemented method of Clause 1, wherein, when the head tilt angle is in a nodding direction, and when the estimated segment is at a front of the dental arch, the orientation of the toothbrush is an amount of roll of the toothbrush, wherein, when the head tilt angle is in a nodding direction, and when the estimated segment is at a side of the dental arch, the orientation of the toothbrush is an amount of pitch of the toothbrush, wherein, when the head tilt angle is in a sideways rolling direction, and when the estimated segment is at a front of the dental arch, the orientation of the toothbrush is an amount of pitch of the toothbrush, and wherein, when the head tilt angle is in a sideways rolling direction, and when the estimated segment is at a side of the dental arch, the orientation of the toothbrush is an amount of roll of the toothbrush.

Clause 3. The computer-implemented method of Clause 1, wherein the machine learning model is a second machine learning model, and wherein the method further comprises: inputting, to a first machine learning model, the sensed movement parameters to estimate a brushing angle between bristles at a head of the toothbrush and teeth of the user at each time point of the brushing time period, and wherein the, for each time point, calculating the head tilt angle of the user of the toothbrush based on the estimated segment of the dental arch and the calculated orientation comprises: subtracting the calculated orientation from the brushing angle.

Clause 4. The computer-implemented method of Clause 3, wherein the first and the second machine learning models each comprises a long short-term memory recurrent neural network, LSTM.

Clause 5. The computer-implemented method of Clause 4, wherein the first machine learning model comprises an encoder between an input layer and the LSTM and comprises a decoder between the LSTM and an output layer, wherein the encoder comprises a convolutional neural network, wherein the decoder comprises a convolutional neural network.

Clause 6. The computer-implemented method of Clause 5, wherein the encoder and the LSTM of the first machine learning model is shared with the second machine learning model.

Clause 7. The computer-implemented method of Claim 1, wherein the movement parameters include acceleration and angular velocity and the sensor is an inertial measurement unit.

Clause 8. The computer-implemented method of Claim 1, movement parameters to estimate a segment of the dental arch being contacted by the bristles at each time point of the brushing time period comprises inputting, to the second machine learning model, the sensed movement parameters to estimate a segment of the dental arch being contact by the bristles from amongst a list a segments of dental arches including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual.

Clause 9. A computer-implemented method of training a machine learning model to estimate a segment of a dental arch of a user being brushed by a user of a toothbrush, the computer-implemented method comprising: receiving a training data set and a segment ground truth, wherein, for each time point of a brushing time period, the training data set includes movement parameter values and the segment ground truth includes a label indicating a segment of a dental arch being brushed; estimating, using the machine learning model, a brushing segment for each time point of the brushing time period using the respective movement parameter values as inputs; calculating a loss between the estimated brushing segments and the segments indicated by the labels; and adjusting a parameterisation of the machine learning model to minimize the loss.

Clause 10. The computer-implemented method of Clause 9, further comprising: generating the segment ground truth by: capturing a video of a user brushing their teeth using a toothbrush over a sample period, sensing, using a sensor of the toothbrush, movement parameter values at each time point of the sample period, displaying the video to a user of a user interface, receiving a plurality of user inputs indicating a segment of a dental arch currently being brushed by the toothbrush in the video, and labelling each time point of the sample period using the segments from the plurality of user inputs; and sending the ground truth to the machine learning model.

Clause 11. A computer-implemented method of training a machine learning model to estimate a brushing angle between bristles of a toothbrush and teeth of a user of the toothbrush, the computer-implemented method comprising: receiving a training data set and a brushing angle ground truth, wherein, for each time point of a brushing time period, the training data set includes movement parameter values and the brushing angle ground truth; estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss.

Clause 12. The computer-implemented method of Clause 11, further comprising: generating the brushing angle ground truth by: capturing a video of a user brushing their teeth with a toothbrush over a sample period, the user having a first marker on their forehead and the toothbrush having a second marker on its handle, sensing, using a sensor of the toothbrush, movement parameter values at each time point of the sample period, calculating, at each time point of the sample period, a brushing angle based on relative positions of the first marker and the second marker, and generating, for each time point, a brushing angle ground truth associated with the movement parameter values; and sending the brushing angle ground truth to the machine learning model.

Clause 13. The computer-implemented method of Clause 12, wherein the calculating a brushing angle based on relative positions of the first marker and the second marker comprises calculating the brushing angle using formula 1 for upper teeth and using formula 2 for lower teeth, wherein formula 1 is $\beta = arctan2((h. n), - (h. z))$, and formula 2 is a $\beta = arctan2((h. n), (h. z))$, where $\beta$ is the brushing angle, h denotes a vector pointing away from a point P along bristles of the toothbrush to the toothbrush, where P denotes a point where the bristles touch the teeth, n denotes a vector extending outwardly normal to a curve of the teeth, and z denotes an upward, in-use, normal to a plane of an upper jaw.

Clause 14. The computer-implemented method of Clause 12, wherein the calculating a loss between the estimated brushing angles and the brushing angle ground truth comprises, for each time point of the brushing time period, determining a ground truth unit vector, vgt, and an estimated brushing angle unit vector, veba, by respectively calculating cosine and sine components of the brushing angles in the ground truth and the estimated brushing angles, and calculating the loss, L, using $L = 1 - v_{gt}. v_{eba}$, where . denotes an inner dot product between vgt and veba.

Clause 15. The computer-implemented method of Clause 11, further comprising: training a machine learning model to estimate a segment of a dental arch of a user being brushed by a user of a toothbrush, the computer-implemented method comprising: receiving a training data set and a segment ground truth, wherein, for each time point of a brushing time period, the training data set includes movement parameter values and the segment ground truth includes a label indicating a segment of a dental arch being brushed; estimating, using the machine learning model, a brushing segment for each time point of the brushing time period using the respective movement parameter values as inputs; calculating a loss between the estimated brushing segments and the segments indicated by the labels; and adjusting a parameterisation of the machine learning model to minimize the loss; re-initialising parameters associated with a final layer of the machine learning model; and fine-tuning the machine learning model by the: estimating, using the machine learning model, a brushing angle for each time point of the brushing time period using the respective movement parameter values as inputs; calculating a loss between the estimated brushing angles and the brushing angle ground truth; and adjusting a parameterisation of the machine learning model to minimise the loss.

## Claims

1. A computer-implemented method of estimating a head tilt angle of a user of a toothbrush during brushing, the computer-implemented method comprising:

   sensing (S100), using a sensor (20) of the toothbrush (10), movement parameters associated with the toothbrush over a brushing time period;
   inputting (S102), to a machine learning model (60), the sensed movement parameters to estimate a segment of a dental arch (30, 32) being contacted by the bristles (26) at each time point of the brushing time period;
   calculating (S104), using a mathematical heuristics model (74), an orientation of the toothbrush (10) from the movement parameters at each time point of the brushing time period; and
   for each time point, calculating (S106) the head tilt angle of the user of the toothbrush based on the estimated segment of the dental arch (30, 32) and the calculated orientation.

2. The computer-implemented method of Claim 1, wherein, when the head tilt angle is in a nodding direction, and when the estimated segment is at a front of the dental arch, the orientation of the toothbrush is an amount of roll of the toothbrush,

> wherein, when the head tilt angle is in a nodding direction, and when the estimated segment is at a side (44, 46) of the dental arch, the orientation of the toothbrush is an amount of pitch of the toothbrush,
> wherein, when the head tilt angle is in a sideways rolling direction, and when the estimated segment is at a front (42) of the dental arch, the orientation of the toothbrush is an amount of pitch of the toothbrush, and
> wherein, when the head tilt angle is in a sideways rolling direction, and when the estimated segment is at a side of the dental arch, the orientation of the toothbrush is an amount of roll of the toothbrush.

3. The computer-implemented method of Claim 1 or Claim 2, wherein the machine learning model is a second machine learning model, and wherein the method further comprises:

> inputting, to a first machine learning model (50), the sensed movement parameters to estimate a brushing angle between bristles at a head of the toothbrush and teeth of the user at each time point of the brushing time period, and
> wherein the, for each time point, calculating the head tilt angle of the user of the toothbrush based on the estimated segment of the dental arch and the calculated orientation comprises:
> subtracting the calculated orientation from the brushing angle.

4. The computer-implemented method of Claim 3, wherein the first and the second machine learning models each comprises a long short-term memory recurrent neural network, LSTM, (53).

5. The computer-implemented method of Claim 4, wherein the first machine learning model comprises an encoder (52) between an input layer (51) and the LSTM and comprises a decoder (54) between the LSTM and an output layer (55), wherein the encoder comprises a convolutional neural network (56), wherein the decoder comprises a convolutional neural network (58).

6. The computer-implemented method of Claim 5, wherein the encoder and the LSTM of the first machine learning model is shared with the second machine learning model.

7. The computer-implemented method of any preceding claim, wherein the movement parameters include acceleration and angular velocity and the sensor is an inertial measurement unit.

8. The computer-implemented method of any preceding claim, movement parameters to estimate a segment of the dental arch being contacted by the bristles at each time point of the brushing time period comprises inputting, to the second machine learning model, the sensed movement parameters to estimate a segment of the dental arch being contact by the bristles from amongst a list a segments of dental arches including upper front facial, and upper front lingual, upper right facial, upper right occlusal, upper right lingual, upper left facial, upper left occlusal, upper left lingual, lower front facial, and lower front lingual, lower right facial, lower right occlusal, lower right lingual, lower left facial, lower left occlusal, lower left lingual.

9. A computer-implemented method of training a machine learning model (60) to estimate a segment of a dental arch (30, 32) of a user being brushed by a user of a toothbrush (10), the computer-implemented method comprising:

> receiving (S200) a training data set and a segment ground truth, wherein, for each time point of a brushing time period, the training data set includes movement parameter values and the segment ground truth includes a label indicating a segment of a dental arch being brushed;
> estimating (S202), using the machine learning model, a brushing segment for each time point of the brushing time period using the respective movement parameter values as inputs;
> calculating (S204) a loss between the estimated brushing segments and the segments indicated by the labels; and
> adjusting (S206) a parameterisation of the machine learning model to minimize the loss.

10. The computer-implemented method of Claim 9, further comprising:
generating the segment ground truth by:

> capturing a video of a user brushing their teeth using a toothbrush over a sample period,

sensing, using a sensor of the toothbrush, movement parameter values at each time point of the sample period,

displaying the video to a user of a user interface,

receiving a plurality of user inputs indicating a segment of a dental arch currently being brushed by the toothbrush in the video, and

labelling each time point of the sample period using the segments from the plurality of user inputs; and sending the ground truth to the machine learning model.

11. A computer-implemented method of training a machine learning model to estimate a brushing angle between bristles (26) of a toothbrush and teeth of a user of the toothbrush (10), the computer-implemented method comprising:

receiving (S300) a training data set and a brushing angle ground truth, wherein, for each time point of a brushing time period, the training data set includes movement parameter values and the brushing angle ground truth;

estimating (S302), using the machine learning model, a brushing angle for each time point of the brushing time period using the respective movement parameter values as inputs;

calculating (S304) a loss between the estimated brushing angles and the brushing angle ground truth; and

adjusting (S306) a parameterisation of the machine learning model to minimise the loss.

12. The computer-implemented method of Claim 11, further comprising:

generating the brushing angle ground truth by:

capturing a video of a user brushing their teeth with a toothbrush over a sample period, the user having a first marker on their forehead and the toothbrush having a second marker on its handle,

sensing, using a sensor of the toothbrush, movement parameter values at each time point of the sample period,

calculating, at each time point of the sample period, a brushing angle based on relative positions of the first marker and the second marker, and

generating, for each time point, a brushing angle ground truth associated with the movement parameter values; and

sending the brushing angle ground truth to the machine learning model.

13. The computer-implemented method of Claim 12, wherein the calculating a brushing angle based on relative positions of the first marker and the second marker comprises calculating the brushing angle using formula 1 for upper teeth and using formula 2 for lower teeth,

wherein formula 1 is $\beta = arctan2\,((h.\,n),\,-(h.z))$, and formula 2 is a $\beta = arctan2\,((h.\,n),\,(h.\,z))$, where $\beta$ is the brushing angle, h denotes a vector pointing away from a point P along bristles of the toothbrush to the toothbrush, where P denotes a point where the bristles touch the teeth, n denotes a vector extending outwardly normal to a curve of the teeth, and z denotes an upward, in-use, normal to a plane of an upper jaw.

14. The computer-implemented method of any of Claims 12 to 13, wherein the calculating a loss between the estimated brushing angles and the brushing angle ground truth comprises, for each time point of the brushing time period, determining a ground truth unit vector, $v_{gt}$, and an estimated brushing angle unit vector, $v_{eba}$, by respectively calculating cosine and sine components of the brushing angles in the ground truth and the estimated brushing angles, and calculating the loss, L, using $L = 1 - v_{gt}.v_{eba}$, where . denotes an inner dot product between $v_{gt}$ and $v_{eba}$.

15. The computer-implemented method of any of Claims 11 to 14, further comprising:

providing the machine learning model trained using the computer-implemented method of any of Claims 9 to 10;

re-initialising parameters associated with a final layer of the machine learning model; and

fine-tuning the machine learning model by the:

estimating, using the machine learning model, a brushing angle for each time point of the brushing time period using the respective movement parameter values as inputs;

calculating a loss between the estimated brushing angles and the brushing angle ground truth; and

adjusting a parameterisation of the machine learning model to minimise the loss.

10

12

14

18

22    24    16    20

26

Figure 1

30

34
35
36
37
37

42

46    44    38
38
38

32

38
38
38
37

37

36
35

46    44

42

34

Figure 2

upper teeth, brushing angle
$$\arctan2\big((h \cdot n), -(h \cdot z)\big).$$

lower teeth, brushing angle
$$\arctan2\big((h \cdot n), (h \cdot z)\big).$$

Figure 3

Figure 4

session_0005, loss = 0.0117388 mae = 7.113

Figure 5A

session_0006, loss = 0.00959113 mae = 6.17032

Figure 5B

Figure 6

Figure 7

74

Figure 8

No head tilt    Head tilt present

Figure 9

Figure 10

Figure 11

Figure 12

S100

sensing, using a sensor of the toothbrush, movement parameters associated with the toothbrush over a brushing time period

S102

inputting, to a machine learning model, the sensed movement parameters to estimate a segment of a dental arch being contacted by the bristles at each time point of the brushing time period

S104

calculating, using a mathematical heuristics model, an orientation of the toothbrush from the movement parameters at each time point of the brushing time period

S106

for each time point, calculating the head tilt angle of the user of the toothbrush based on the estimated segment of the dental arch and the calculated orientation

Figure 13

S200

receiving a training data set and a segment ground truth, wherein, for each time point of a brushing time period, the training data set includes movement parameter values and the segment ground truth includes a label indicating a segment of teeth being brushed

S202

estimating, using the machine learning model, a brushing segment for each time point of the brushing time period using the respective movement parameter values as inputs

S204

calculating a loss between the estimated brushing segments and the segments indicated by the labels

S206

adjusting a parameterisation of the machine learning model to minimize the loss

Figure 14

S300

receiving a training data set and a brushing angle ground truth, wherein, for each time point of a brushing time period, the training data set includes movement parameter values and the brushing angle ground truth

S302

estimating, using the machine learning model, a brushing angle for each time point of the brushing time period using the respective movement parameter values as inputs

S304

calculating a loss between the estimated brushing angles and the brushing angle ground truth

S306

adjusting a parameterisation of the machine learning model to minimise the loss

Figure 15

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 23 18 3884

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2010/323337 A1 (IKKINK TEUNIS JAN [NL] ET AL) 23 December 2010 (2010-12-23) * paragraphs [0006], [0012], [0013], [0018] – [0020], [0026], [0027] – [0031]; figures 1,4 * | 1-15 | INV. A46B15/00 A61B5/107 |
| Y | US 2022/249214 A1 (SERVAL THOMAS [FR] ET AL) 11 August 2022 (2022-08-11) * paragraphs [0198], [0199], [0207]; figures 1,2 * | 1-15 | |
| Y | US 2020/022488 A1 (DEN HAMER ARJEN [NL] ET AL) 23 January 2020 (2020-01-23) * paragraphs [0028] – [0033]; figures 1,3 * | 1 | |
| Y | CN 105 029 891 A (QIN LIXIN) 11 November 2015 (2015-11-11) * claims 1-5,11 * | 1 | |
| A | WO 2022/090184 A1 (KONINKLIJKE PHILIPS NV [NL]) 5 May 2022 (2022-05-05) * paragraph [0065] * | 1-15 | TECHNICAL FIELDS SEARCHED (IPC) A46B |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| The Hague | 7 March 2024 | Rossini, Marco |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................
& : member of the same patent family, corresponding
   document

EPO FORM 1503 03.82 (P04E07)

**INCOMPLETE SEARCH
SHEET C**

```
Claim(s) searched incompletely:
      1-15

Reason for the limitation of the search (non-patentable invention(s)):

Article 53 (c) EPC - Method for treatment of the human or animal body by
therapy
```

## ANNEX TO THE EUROPEAN SEARCH REPORT
## ON EUROPEAN PATENT APPLICATION NO.

EP 23 18 3884

This annex lists the patent family members relating to the patent documents cited in  the above-mentioned European search report.
The members are as contained in the European Patent Office EDP file on
The European Patent Office is in no way liable for these particulars which are merely given for the purpose of information.

07-03-2024

| Patent document cited in search report | Publication date | Patent family member(s) | Publication date |
|---|---|---|---|
| US 2010323337 A1 | 23-12-2010 | BR PI0907548 A2 | 28-07-2015 |
| | | CA 2717092 A1 | 03-09-2009 |
| | | CN 102065791 A | 18-05-2011 |
| | | EP 2244660 A1 | 03-11-2010 |
| | | JP 5587796 B2 | 10-09-2014 |
| | | JP 2011512933 A | 28-04-2011 |
| | | KR 20110002011 A | 06-01-2011 |
| | | RU 2010139399 A | 10-04-2012 |
| | | US 2010323337 A1 | 23-12-2010 |
| | | US 2014246049 A1 | 04-09-2014 |
| | | WO 2009107047 A1 | 03-09-2009 |
| US 2022249214 A1 | 11-08-2022 | AU 2017316731 A1 | 21-03-2019 |
| | | AU 2023200463 A1 | 02-03-2023 |
| | | BR 112019003640 A2 | 21-05-2019 |
| | | CN 110213980 A | 06-09-2019 |
| | | EP 3493701 A1 | 12-06-2019 |
| | | RU 2019106205 A | 22-09-2020 |
| | | US 2020179089 A1 | 11-06-2020 |
| | | US 2022249214 A1 | 11-08-2022 |
| | | US 2023329850 A1 | 19-10-2023 |
| | | WO 2018037318 A1 | 01-03-2018 |
| US 2020022488 A1 | 23-01-2020 | CN 110139582 A | 16-08-2019 |
| | | CN 117224262 A | 15-12-2023 |
| | | EP 3547874 A1 | 09-10-2019 |
| | | JP 2019536560 A | 19-12-2019 |
| | | KR 20190089950 A | 31-07-2019 |
| | | RU 2019120394 A | 11-01-2021 |
| | | US 2020022488 A1 | 23-01-2020 |
| | | WO 2018100121 A1 | 07-06-2018 |
| CN 105029891 A | 11-11-2015 | NONE | |
| WO 2022090184 A1 | 05-05-2022 | CN 116583205 A | 11-08-2023 |
| | | EP 4236864 A1 | 06-09-2023 |
| | | US 2023397713 A1 | 14-12-2023 |
| | | WO 2022090184 A1 | 05-05-2022 |

EPO FORM P0459

For more details about this annex : see Official Journal of the European Patent Office, No. 12/82